# EUROPEAN PATENT SPECIFICATION

(11) **EP 3 014 905 B1**
(45) Date of publication and mention of the grant of the patent: **02.01.2019**
(21) Application number: 14817172.1
(22) Date of filing: 18.06.2014
(51) Int. Cl.: H04L 29/08, H04W 4/02, A63B 24/00, A61B 5/103, A61B 5/11, A61B 5/00

(54) **USER ACTIVITY TRACKING SYSTEM AND DEVICE**
SYSTEM UND VORRICHTUNG ZUR VERFOLGUNG DER BENUTZERAKTIVITÄT
SYSTÈME DE SUIVI DES ACTIVITÉS D'UN UTILISATEUR ET DISPOSITIF

(30) Priority: 28.06.2013 US 201313930321; 28.06.2013 US 201313930347
(43) Date of publication of application: 04.05.2016
(73) Proprietor: Facebook, Inc., Menlo Park, CA 94025 (US)
(72) Inventor: PENNANEN, Juho, Menlo Park, CA 94025 (US); KYRÕLÃ , Aapo, Menlo Park, CA 94025 (US); PARTANEN, Jukka, Menlo Park, CA 94025 (US)
(74) Representative: Schröer, Gernot H.
(86) International application number: PCT/US2014/042839
(87) International publication number: WO 2014/209697

(56) References cited:
- WO-A1-2012/134797
- JP-A- 2009 217 378
- KR-A- 20060 037 640
- KR-A- 20080 013 602
- KR-A- 20090 107 161
- US-A1- 2005 065 443
- US-A1- 2010 131 443
- US-A1- 2011 051 665
- US-A1- 2011 066 383
- US-A1- 2012 326 873
- US-B1- 8 306 514

## Description

### TECHNICAL FIELD

The present disclosure generally relates to tracking, recording and analyzing user activities, and more specifically to systems and associated methods for identifying user activities based on sensor data collected by a mobile communication device of a user. Moreover, aspects of the disclosure are also directed to software products recorded on non-transitory machine-readable data storage media, wherein such software products are executable upon computing hardware, to implement the methods of the disclosure.

### BACKGROUND

Tracking devices exist that sense and track user activities, especially sports activities. An example of a known activity tracking device is a wearable wristwatch device with a GPS receiver for tracking and analyzing 'running' activity of an associated user. Another example is a mobile application that utilizes GPS system of a respective mobile phone for recording movement of users while they exercise. Another example is a step counter used in shoes or attached to the user's clothes to collect the number of steps taken by the user. However, none of the existing tracking devices automatically sense, record, analyze and identify all types of user activities such as walking, running, jogging, cycling, rowing, driving with car, moving with bus, moving with train, walking stairs, running stairs, jumping, swimming, playing football, and skiing.

Nowadays, smartphones are equipped with an increasing number of sensors such as Global Positioning System (GPS) receivers, accelerometers, and proximity sensors, and smartphone users may find it interesting to have mobile applications that can automatically record, sense, analyze, and identify their activities. However, one of the key challenges in the automatic tracking of users' movements for the purpose of analyzing the type of activity is the classification of activity types. For example walking vs running activity may have only small difference in respect to the collected sensor data. Moreover, for the same activity, the sensor data may vary depending on how the smart phone is carried by the user. For example, the smartphone may be carried by the user in his/her hand, or in a pocket or in a backpack.

Hence, there exists a need for an activity tracking solution that accurately senses and analyzes all kinds of user activities and that addresses the limitations of existing activity tracking solutions.

In US 2011/0066383 A1, methods, systems and an apparatus for identifying an activity of an animate or inanimate object are disclosed. One method includes identifying each elemental motion of a sequence of elemental motions of a device attached to the animate or inanimate object. The activity of the animate or inanimate object can be identified by matching the sequence of identified elemental motions of the device with a library of stored sequences of elemental motions, wherein each stored sequence of elemental motions corresponds with an activity. Similarly, WO 2012/134797 discloses activity classification using temporal scaling of time-referenced features of received sensor signals, from which user activities could be inferred. In an embodiment, the process of classifying user activity is carried-out using temporal scaling of time-referenced features extracted from a plurality of movement sensors of a mobile device.

US 2005/0065443 A1 discusses, among other things, cardiac rhythm management systems, devices, and methods providing an exercise test interface. A patient exercise episode is identified using at least one predetermined criteria. Data associated with the episode is obtained. A summary of the episode is displayed for a user. The displayed summary includes at least one prognostic indicator obtained from the data associated with the episode. Examples of prognostic indicators include, among other things, ectopic beats, runs of ectopic beats, rate of decrease of heart rate during a post-exercise recovery portion of the episode, a maximum attained heart rate for comparison to a maximum age-predicted heart rate, as well as other prognostic indicators.

### SUMMARY OF PARTICULAR EMBODIMENTS

The present disclosure seeks to provide a system for tracking and recording movements of a mobile communication device and a method of the using the same.

The present invention provides a method of operating a system for tracking and recording movements of at least one mobile communication device according to claim 1, a system for tracking and recording movements of at least one mobile communication device according to claim 10, one or more computer-readable non-transitory storage media according to claim 11, a storage media according to claim 12, a mobile communication device according to claim 13 and a computer-implemented method according to claim 14. Further improvements are provided in the dependent claims.

In one aspect, embodiments of the present disclosure provide a system for tracking and recording movements of a mobile communication device that includes one or more movement sensors and a wireless interface. The system includes a communication network for communicating with the mobile communication device and computing hardware for processing data supplied in operation from the mobile communication device. The mobile communication device communicates sensor signals, for example in a form of sensor data, to the system, wherein the sensor signals are indicative of motion associated with activities to which the mobile communication device is exposed by its user.

In particular embodiments, the computing hardware executes software products for analyzing the sensor signals to classify them into one or more temporal zones, and for analyzing the signals within each given temporal zone to determine one or more most likely activity types associated with the given temporal zone. The computing hardware further sends information indicating the most likely activity types associated with the temporal zones to the mobile communication device. The mobile communication device then requests its user to provide a confirmation whether or not the information indicating the most likely activity types associated with a temporal zone represents a correct analysis, and then communicates the confirmation back to the computing hardware for amending parameters and/or algorithms employed in the software products, which execute analysis of the sensor signals to improve their accuracy.

In particular embodiments, the computing hardware executes software products for analyzing the sensor signals to pre-classify the sensor signals to generate intermediate data. The intermediate data is thereafter processed in one or more processors to generate indications of likely activities associated with the sensor signals. The computing hardware further computes an aggregate of the indications to provide an analysis of one or more activities associated with the sensor signals, and then sends information indicating most likely activity types to the mobile communication device.

The processors are configured to process the sensor signals substantially in parallel, wherein the processors are mutually specialized in identifying characteristics of the signals corresponding to activities to which the processors are dedicated.

The system generates a temporal log of activities experienced by the mobile communication device, and presents the activities on a graphical user interface of a user in a timeline format.

The mobile communication device is implemented by way of at least one of: a portable computer such as laptop, a smartphone, a wrist-worn phone, a phablet, a mobile telephone, a tablet computer, a portable media device or any other computing device that can be worn by the user and is capable of processing and displaying data. Further, one or more sensors of the mobile communication device are implemented using at least one of: a gyroscopic angular sensor, an accelerometer, a GPS position sensor, cellular positioning sensor, a magnetometer, a microphone, a camera, a temperature sensor. The term cellular positioning sensor can refer to determining the location and movement of the mobile communication device can be derived/analyzed/measured using information related to a cellular network and information related to radio base stations and their signals.

When executed on the computing hardware, the software products are operable to implement supervised or semisupervised classification algorithms such as neural networks, decision forest, and support vector machines, for analysis of information included in the sensor signals. As input, the supervised or semisupervised classification algorithms can use, for instance, the amplitudes of the frequency components of the information included in the one or more sensor signals, and the output of the classification algorithms are estimated probabilities of different activities, conditional on the sensor signals.

In another aspect, the mobile communication device includes a data processor for executing a mobile software application thereat, wherein the mobile software application is operable when executed to cause a graphical user interface of the mobile communication device to present analyzed activity results provided from the computing hardware in a form of a timeline, wherein different analyzed activities are represented by mutually different symbols in respect of the timeline.

In yet another aspect, embodiments of the present disclosure provide a method of using the system for tracking and recording the movements of the mobile communication device.

In yet another aspect, embodiments of the present disclosure provide a mobile communication device for implementing the system for tracking and recording movements of the user.

In yet another aspect, embodiments of the present disclosure provide a software product recorded on a non-transitory machine-readable data storage media, such that the software product is executable upon computing hardware for implementing the method of using the system for tracking and recording movements of the mobile communication device. The software product is downloadable from a software application store to the mobile communication device.

Embodiments of the present disclosure sense, analyze and identify all types of user activities by analyzing the data collected from one or more sensors of a mobile communication device of a user. The sensor data is processed by a set of independent instances of classification algorithms and each instance is optionally dedicated to identify a specific type of activity . The output of the set of classification algorithm instances is aggregated and analyzed to generate most likely user activities associated with the mobile communication device. The identified activities are displayed on a graphical user interface of the mobile communication device in a timeline format. If the user disagrees/agrees with an identified activity, then they may provide their feedback and the feedback may be used to improve the analysis and identification of the activities for the next time. Thus, the accuracy of the analysis and identification of the user activities is optimized over time.

Alternatively embodiments of the present disclosure accurately sense, analyze and identify user activities by analyzing data collected from one or more sensors of a mobile communication device of a user. The sensor data is processed by a set of parallel processors, wherein the parallel processors are parallel instances of classification algorithms and each processor is optionally dedicated to identify a specific type of activity. The output of the set of parallel processors is aggregated and analyzed to generate most likely user activities associated with the mobile communication device. The identified activities are then displayed on a graphical user interface of the mobile communication device in a timeline format. In current disclosure parallel processors can refer to implementation architecture where part of the software is executed in different central processing units (i.e. microprocessors) and/or parallel instances of classification algorithms i.e. in parallel software processes. Parallel can refer to calculation processes executed substantially at the same time but is not limited to such a method. Executing of instances can take place one by one or as a combination of some processes executed substantially at the same time and some processes executed one by one.

Additional aspects, advantages, features and objects of the present disclosure would be made apparent from the drawings and the detailed description of the illustrative embodiments construed in conjunction with the appended claims that follow.

It will be appreciated that features of the invention are susceptible to being combined in various combinations without departing from the scope of the invention as defined by the appended claims.

Embodiments according to the invention are in particular disclosed in the attached claims directed to a method, a storage medium and a system, wherein any feature mentioned in one claim category, e.g. method, can be claimed in another claim category, e.g. system, as well. The dependencies or references back in the attached claims are chosen for formal reasons only. However any subject matter resulting from a deliberate reference back to any previous claims (in particular multiple dependencies) can be claimed as well, so that any combination of claims and the features thereof is disclosed and can be claimed regardless of the dependencies chosen in the attached claims.

In an embodiment according to the invention a method of using a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, characterized in that the method includes:
operating the at least one mobile communication device to communicate one or more sensor signals to the system, wherein the one or more sensor signals are indicative of motion associated with activities to which the at least one mobile communication device is exposed by its user;
operating the computing hardware of the system to execute one or more software products for analyzing the one or more sensor signals, wherein the computing hardware is operable to pre-classify the one or more sensor signals to generate intermediate data, and the intermediate data is thereafter processed in one or more processors to generate one or more indications of likely activities associated with the one or more sensor signals, and the computing hardware is operable to compute an aggregate of the one or more indications to provide an analysis of one or more activities associated with the one or more signals; and
operating the computing hardware to send information indicating one or more most likely activity types to the at least one mobile communication device.

In a further embodiment according to the invention, the method can be characterized in that the method further includes:
operating the computing hardware of the system to execute one or more software products for analyzing the one or more sensor signals to classifying them into one or more temporal zones, and for analyzing the one or more sensor signals within each given temporal zone to determine a most likely activity type associated with the given temporal zone;
operating the computing hardware to send information indicating one or more most likely activity types associated with the one or more temporal zones to the at least one mobile communication device; and
operating the at least one mobile communication device to request its user to provide a confirmation whether or not the information indicating the one or more most likely activity types associated with the one or more temporal zones represent a correct analysis, and to communicate the confirmation back to the computing hardware for amending parameters and/or algorithms employed in the one or more software products which execute analysis of the one or more sensor signals to improve their accuracy.

In a further embodiment according to the invention, the method can be characterized in that the method includes configuring the one or more processors to process the one or more sensor signals substantially in parallel, wherein the one or more processors are mutually specialized in identifying characteristics of the one or more signals corresponding to one or more activities to which the one or more processors are dedicated.

In a further embodiment according to the invention, the method can be characterized in that the method includes operating the system to generate a temporal log of activities experienced by the at least one mobile communication device.

In a further embodiment according to the invention, the method can be characterized in that the method includes implementing at least one mobile communication device by way of at least one of: a personal computer, a portable media device, a smart phone, a wrist-worn phone, a phablet, a mobile telephone, a tablet computer.

In a further embodiment according to the invention, the method can be characterized in that the method includes implementing the one or more sensors of the at least mobile communication device using at least one of: a gyroscopic angular sensor, an accelerometer, a GPS position sensor, a cellular position sensor, a magnetometer, a microphone, a camera, a temperature sensor.

In a further embodiment according to the invention, the method can be characterized in that the one or more software products are operable to implement when executed on the computing hardware at least one of:
a supervised or semisupervised classification analysis of information included in the one or sensor signals; and
a heuristics analysis of information included in the one or more sensor signals.

In a further embodiment according to the invention, the method can be characterized in that the supervised or semisupervised classification algorithms can use as input the amplitudes of the frequency components of the information included in the one or more sensor signals, and the output of the classification algorithms are estimated probabilities of different activities, conditional on the sensor signals.

In a further embodiment according to the invention, the method can be characterized in that the method includes arranging for the at least one mobile communication device to include a data processor for executing at least one mobile software application thereat, wherein the at least one mobile software application is operable to cause a graphical user interface of the at least one mobile communication device to present analyzed activity results provided from the computing hardware in a form of at least one timeline, wherein different analyzed activities are represented by a plurality of mutually different symbols in respect of the at least one time line.

In a further embodiment according to the invention, which can be claimed as well, a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, can be characterized in that
the at least one mobile communication device is operable to communicate one or more sensor signals to the system, wherein the one or more sensor signals are indicative of motion associated with activities to which the at least one mobile communication device is exposed by its user;
the computing hardware of the system is operable to execute one or more software products for analyzing the one or more sensor signals, wherein the computing hardware is operable to pre-classify the one or more sensor signals to generate intermediate data, and the intermediate data is thereafter processed in one or more processors to generate one or more indications of likely activities associated with the one or more sensor signals, and the computing hardware is operable to compute an aggregate of the one or more indications to provide an analysis of one or more activities associated with the one or more signals; and
the computing hardware is operable to send information indicating one or more most likely activity types to the at least one mobile communication device.

In a further embodiment according to the invention, a system can further be characterized in that the computing hardware of the system is operable to execute one or more software products for analyzing the one or more sensor signals to classifying them into one or more temporal zones, and for analyzing the one or more sensor signals within each given temporal zone to determine a most likely activity type associated with the given temporal zone;
the computing hardware is operable to send information indicating one or more most likely activity types associated with the one or more temporal zones to the at least one mobile communication device; and
the at least one mobile communication device is operable to request its user to provide a confirmation whether or not the information indicating the one or more most likely activity types associated with the one or more temporal zones represent a correct analysis, and to communicate the confirmation back to the computing hardware for amending parameters and/or algorithms employed in the one or more software products which execute analysis of the one or more sensor signals to improve their accuracy.

In a further embodiment according to the invention, a system can further be characterized in that the one or more processors are configured to process the one or more sensor signals substantially in parallel, wherein the one or more processors are mutually specialized in identifying characteristics of the one or more signals corresponding to one or more activities to which the one or more processors are dedicated.

In a further embodiment according to the invention, a system can further be characterized in that the system is operable to generate a temporal log of activities experienced by the at least one mobile communication device.

In a further embodiment according to the invention, a system can further be characterized in that the at least one mobile communication device is implemented by way of at least one of: a personal computer, a portable media device, a smart phone, a wrist-worn phone, a phablet, a mobile telephone, a tablet computer.

In a further embodiment according to the invention, a system can further be characterized in that one or more sensors of the at least mobile communication device are implemented using at least one of: a gyroscopic angular sensor, an accelerometer, a GPS position sensor, cellular positioning sensor , a magnetometer, a microphone, a camera, a temperature sensor.

In a further embodiment according to the invention, a system can further be characterized in that the one or more software products are operable to implement when executed on the computing hardware at least one of:
a supervised or semisupervised classification analysis of information included in the one or sensor signals; and
a heuristic analysis of information included in the one or more sensor signals.

In a further embodiment according to the invention, a system can further be characterized in that the supervised or semisupervised classification algorithms can use as input the amplitudes of the frequency components of the information included in the one or more sensor signals, and the output of the classification algorithms are estimated probabilities of different activities, conditional on the sensor signals.

In a further embodiment according to the invention, a system can further be characterized in that the at least one mobile communication device includes a data processor for executing at least one mobile software application thereat, wherein the at least one mobile software application is operable to cause a graphical user interface of the at least one mobile communication device to present analyzed activity results provided from the computing hardware in a form of at least one timeline, wherein different analyzed activities are represented by a plurality of mutually different symbols in respect of the at least one timeline.

In a further embodiment according to the invention, which can be claimed as well, a software product stored on non-transitory machine-readable data storage media, can be characterized in that the software product is executable upon computing hardware of a mobile communication device for implementing a method according to the invention or any of the above mentioned embodiments.

In a further embodiment according to the invention, a software product can be characterized in that the software product is downloadable from a software application store to a mobile communication device.

A further embodiment according to the invention, which can be claimed as well, can be a mobile communication device for use when implementing the system as according to the invention or any of the above mentioned embodiments.

A further embodiment according to the invention can be a mobile communication configured to be implemented in the system as mentioned above and in use for executing a method as mentioned above.

In a further embodiment according to the invention, one or more computer-readable non-transitory storage media embody software that is operable when executed to perform a method according to the invention or any of the above mentioned embodiments.

In a further embodiment according to the invention, a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, comprises: one or more processors; and at least one memory coupled to the processors and comprising instructions executable by the processors, the processors operable when executing the instructions to perform a method according to the invention or any of the above mentioned embodiments.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the invention is not limited to specific methods and instrumentalities disclosed herein. Moreover, those in the art will understand that the drawings are not to scale. Wherever possible, like elements have been indicated by identical numbers.
Fig. 1 is an illustration of a high-level architecture of a system that is suitable for practicing various implementations of the present disclosure;
Fig. 2 is an illustration of an exchange of information between a mobile communication device and a computing hardware of Fig. 1, in accordance with the present disclosure;
Fig. 3 is an illustration of a graphical user interface (GUI) of the mobile communication device, in accordance with the present disclosure;
Fig. 4 is an illustration of an alternative layout of the GUI of the mobile communication device, in accordance with the present disclosure;
Fig. 5 is an illustration of steps of a method of determining activities of a user of a mobile communication device, in accordance with the present disclosure; and
Fig. 6 is an illustration of steps of using a system for tracking and recording movements of the mobile communication device, in accordance with the present disclosure.

### DESCRIPTION OF EXAMPLE EMBODIMENTS

The following detailed description illustrates embodiments of the disclosure and ways in which it can be implemented. Although the best mode of carrying out the invention has been disclosed, those in the art would recognize that other embodiments for carrying out or practicing the invention are also possible.

The present disclosure provides a system for tracking and recording movements of a mobile communication device that includes one or more movement sensors and a wireless interface. The system includes a communication network for communicating with the mobile communication device and computing hardware for processing data supplied in operation from the mobile communication device. The mobile communication device communicates one or more sensor signals to the system, wherein the sensor signals are indicative of motion associated with activities to which the mobile communication device is exposed by its user.

The computing hardware executes one or more software products for analyzing the sensor signals to classify them into one or more temporal zones, and for analyzing the signals within each given temporal zone to determine the most likely activity type associated with the given temporal zone. The computing hardware further sends information indicating the most likely activity types associated with the temporal zones to the mobile communication device. The mobile communication device then requests its user to provide a confirmation whether or not the information indicating the most likely activity types associated with the temporal zones represent a correct analysis, and then communicates the confirmation back to the computing hardware for amending parameters and/or algorithms employed in the software products that execute analysis of the sensor signals to improve their accuracy.

The computing hardware executes one or more software products for analyzing the sensor signals to pre-classify the sensor signals to generate intermediate data, and the intermediate data is thereafter processed in one or more processors to generate one or more indications of likely activities associated with the sensor signals. The computing hardware further computes an aggregate of the indications to provide an analysis of one or more activities associated with the sensor signals, and sends information indicating most likely activity types to the mobile communication device.

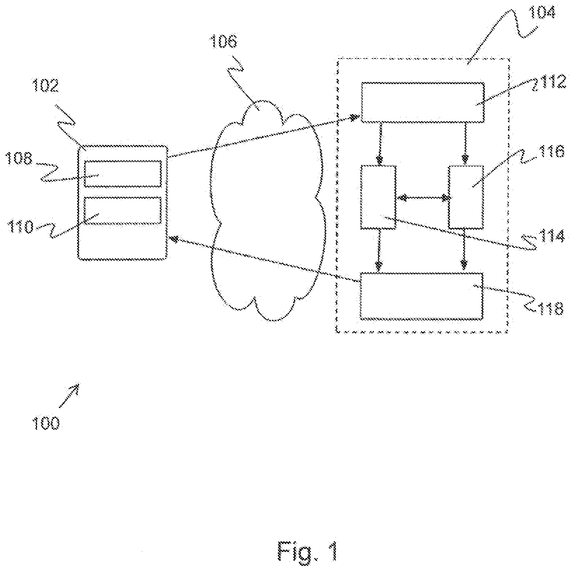

Referring now to the drawings, particularly by their reference numbers, **Fig. 1** is an illustration of a high-level architecture of a system **100** that is suitable for practicing various implementations of the present disclosure.

The system **100** includes a mobile communication device **102,** and a server system **104** coupled in communication to the mobile communication device **102** by way of a communication network **106.** The mobile communication device **102** is a handheld device of a user, and examples of the mobile communication device **102,** include, but are not limited to, a smart phone, a wrist-worn phone, a phablet, a mobile telephone, a tablet computer executing operating systems such as Android, Windows, and iOS. The server system **104** includes a computing hardware that executes one or more software products for processing data supplied in operation from the mobile communication device **102.** The server system **104** can be arranged as a cloud service or as dedicated servers located in a single site or at a plurality of mutually spatially distributed sites. Moreover, examples of the communication network **106** include, but are not limited to, a telecommunication network, and a WIFI network.

The mobile communication device **102** includes one or more sensors **108** and one or more positioning systems **110** to determine the position, movement, acceleration and/or environment of the mobile communication device **102**, when **a** corresponding user performs one or more activities while carrying the device **102.** Examples of such one or more activities, include, but are not limited to, walking, running, jogging, cycling, rowing, driving a car, moving with bus, moving with train, walking stairs, running stairs, jumping, swimming, playing football, and skiing. An example of the sensor **108** includes a motion sensor configured to measure the acceleration of the mobile communication device **102** in xyz-directions of a Cartesian coordinate system. Further examples of the sensor **108** include a gyroscopic angular sensor, a magnetometer, a microphone, a camera, and a temperature sensor. The positioning systems **110** are configured to determine the position of the mobile communication device **102** by implementing at least one of GPS positioning system, cell tower information for cellular networks, indoor positioning systems, WIFI fingerprinting and proximal WiFi networks. In an embodiment of the present invention, the mobile communication device **102** may periodically send the information collected by the sensors **108** and the positioning systems **110** to the server system **104** via the communication network **106**.

The server system **104** includes a receiving module **112,** a first processing module **114,** a second processing module **116**, and an output module **118**. The receiving module **112** receives sensor and positioning data from the mobile communication device **102**. The first processing module **114** executes a first process to analyze sensor data collected from the sensors **108**, and the second processing module **116** executes a second process to analyze positioning data collected from the positioning systems **110**. In an embodiment of the present disclosure, the first and second processes are parallel processes that might communicate with each other and also exchange data for analysis purposes. Based on the sensor data, the first processing module **114** generates an activity type of the user, and based on the positioning data, the second processing module **116** generates location and movement information pertaining to the activity. The output module **118** processes the activity type information and movement/location information of the activity to generate a summary/schedule of activities of the user. The output module **118** then sends the summary of activities to the mobile communication device **102** over the communication network **106.**

The mobile communication device **102** includes a data processor (not shown) for executing a mobile software application thereat, wherein the mobile software application is operable when executed to cause a graphical user interface (GUI) of the mobile communication device to present summary of activities provided from the server system **104** in a timeline format. The user may send their positive/negative feedback on the summary of activities to the server system **104** and the server system **104** may receive, store and implement the feedback for improving their activity analysis.

In an embodiment of the present invention, some or all of the steps/analysis in the server system **104** may be implemented in the mobile communication device **102** based on the computing resources available in the mobile communication device **102.**

Fig. 1 is merely an example, which should not unduly limit the scope of the claims herein. One of ordinary skill in the art would recognize many variations, alternatives, and modifications of embodiments herein.

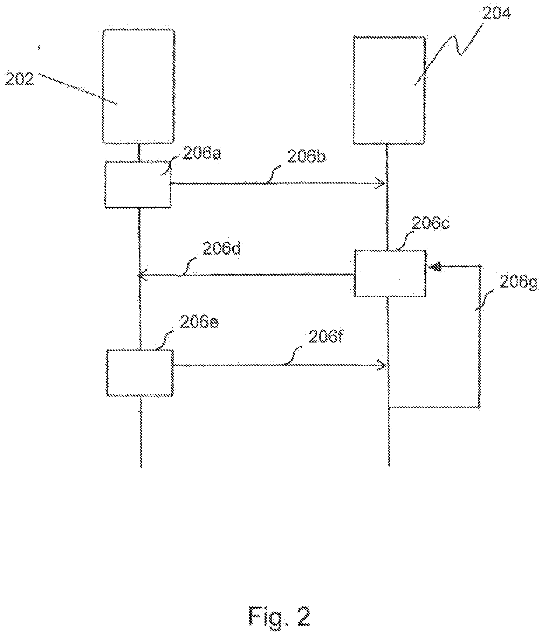

**Fig. 2** is an illustration of an exchange of information between a mobile communication device **202,** and a server system **204** for tracking and analyzing activities of a user of the mobile communication device **202,** in accordance with the present disclosure. The mobile communication device **202** and the server system **204** are examples of the mobile communication device **102** and the server system **104** respectively, and have been explained in conjunction with Fig. 1. A step **206a** takes place at the mobile communication device **202,** where the corresponding sensors and positioning systems measure sensor and positioning data when a corresponding user performs an activity. A step **206b** represents transfer of sensor and positioning data from the mobile communication device **202** to the server system **204** for analysis of the user activity. In an embodiment, the mobile communication device **202** may send the sensor and positioning data continuously or in a buffered format. In another embodiment, the mobile communication device **202** may send the sensor and positioning data to the server system **204** in a raw format. In yet another embodiment, the mobile communication device **202** may process sensor and positioning data before sending to the server system **204.** A step **206c** takes place at the server system **204,** where the server system **204** analyses the sensor and positioning data received from the device **202.** In an embodiment of the present invention, the server system **204** may perform analysis based on a supervised classification and/or other machine learning algorithms and form a summary of activities of the user. A step **206d** represents communication of summary of activities from the server system **204** to the mobile communication device **202.** A step **206e** takes place at the mobile communication device **202,** where the summary of activities is displayed on a graphical user interface (GUI) of the mobile communication device **202.** A step **206f** represents a transfer of positive/negative feedback of the user on the summary of activities to the server system **204.** Lastly, a step **206g** represents implementation of the feedback by the server system **204** by modifying parameters and/or to selecting training data for the machine learning algorithms for providing a more accurate summary of activities in future. Examples of machine learning algorithms, for activity monitoring include, but are not limited to, supervised or semisupervised classification algorithms such as neural networks, decision forest, and support vector machines. The feedback is provided as an input to the machine learning algorithms and is used to modify parameters and select training data for the machine learning algorithms.

Fig. 2 is merely an example, which should not unduly limit the scope of the claims herein. One of ordinary skill in the art would recognize many variations, alternatives, and modifications of embodiments herein.

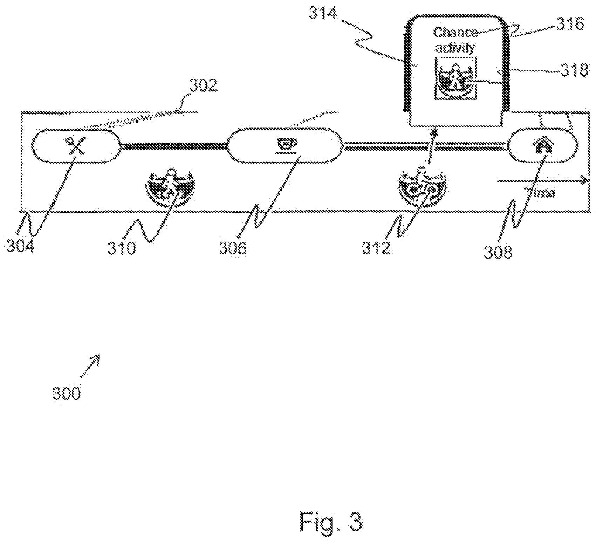

**Fig. 3** is an illustration of a graphical user interface (GUI) **302** of the mobile communication device **200,** in accordance with the present disclosure, and has been explained in conjunction with Fig. 2.

The GUI **302** displays the summary of activities of a user (received from the server system **204** at the step **206d)** in a time line format. In an exemplary embodiment, the GUI **302** displays location of the user by three location symbols, i.e. a restaurant symbol **304,** a cafeteria symbol **306** and a home symbol **308,** indicating that the user has visited the restaurant, cafeteria and home one after the other. The locations represented by the graphical symbols **304, 306** and **308** are identified based on the GPS system of the mobile communication device **202,** cell tower information for cellular networks, indoor positioning systems, proximal WiFi, and/or WiFi fingerprinting. The GUI **302** further displays activities performed by the user by respective graphical symbols. In an example, a 'walking' activity symbol **310** is displayed between the restaurant **304** and cafeteria **306** to indicate that the user has walked from the restaurant to cafeteria. Further, a 'cycling' activity symbol **312** is displayed between the cafeteria **306** and the home **308** to indicate that the user has cycled from the cafeteria to home.

The story line display **302** may be a touch screen display and may be configured in a manner such that when a user touches or points an activity indication symbol such as the 'cycling' activity symbol **312**, or a line above or an area close to the symbol **312**, a pop-up menu **314** is presented. The pop-up menu **314** includes a text field **316** for enabling the user to modify the activity and a symbol field **318** for enabling the user to modify the graphical symbol pertaining to the activity. Similarly when a user touches or points a location symbol such as the 'restaurant' symbol **304**, or a line above or an area close to the symbol **304**, a pop-up menu (not shown) may be presented to modify the location. The modification of location/activity symbol by the user is communicated to the server system **204** as a feedback (step **206f**).

Fig. 3 is merely an example, which should not unduly limit the scope of the claims herein. One of ordinary skill in the art would recognize many variations, alternatives, and modifications of embodiments herein.

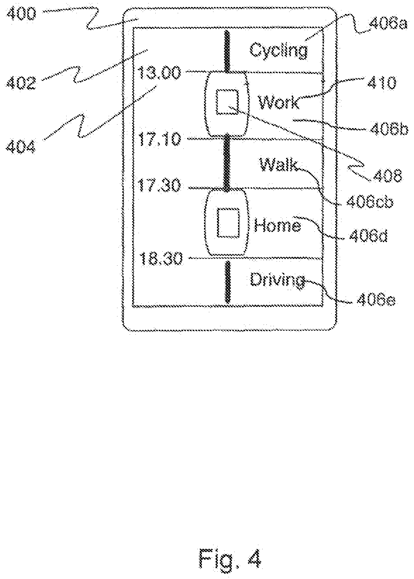

**Fig. 4** is an illustration of a graphical user interface (GUI) **402** of a mobile communication device **400**, which is an example of the mobile communication device **200**, and has been explained in conjunction with Figs. 2 and 3. The GUI **402** is an alternate layout of the GUI **302.** The GUI **402** displays a time line **404** that is divided into 'activity' zones/periods **406a**, **406b, 406c, 406d** and **406e,** hereinafter collectively referred to as activity zones **406**, based on start and end time of one or more activities. Each activity zone **406** illustrates an activity and corresponding location of the activity. Further, each activity zone **406** may be illustrated by a graphical symbol **408** or a text description **410** of the corresponding activity.

In an exemplary embodiment, the timeline **404** indicates that at 13:00 pm, a 'cycling' activity of user ends and he/she is stationed at a 'workplace' until 17:10 pm; at 17:10 pm, the user starts a 'walking' activity towards home; at 17:30 pm, the user reaches home and is at home until 18:30 pm; at 18:30 pm, the user starts a 'driving' activity.

Fig. 4 is merely an example, which should not unduly limit the scope of the claims herein. One of ordinary skill in the art would recognize many variations, alternatives, and modifications of embodiments herein.

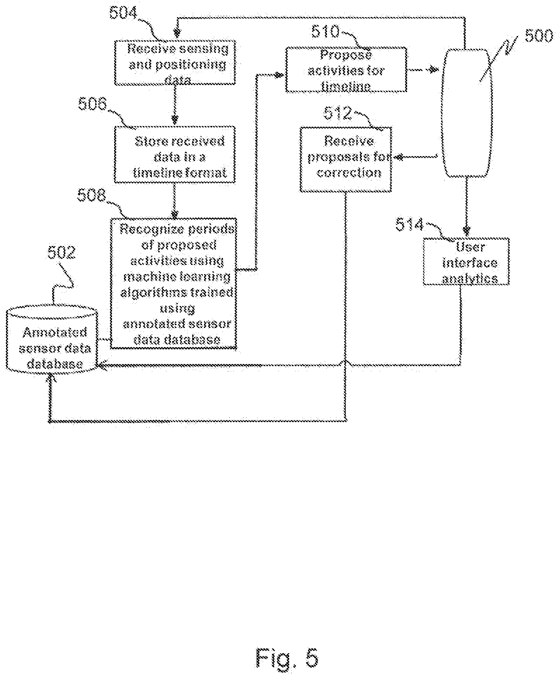

**Fig. 5** is an illustration of steps of a method of determining activities of a user of a mobile communication device **500**, in accordance with the present disclosure. The user activities are determined by an analysis logic module (not shown) which is supplied with the sensor and positioning data generated in operation by the mobile communication device **500**, and an annotated sensor data database **502**. The analysis logic module is communicatively coupled to the annotated sensor data database **502**. In an embodiment of the present invention, the analysis logic module and the annotated sensor data database **502** may be present inside a remote server system. In another embodiment of the present invention, the analysis logic module and the annotated sensor data database **502** may be present inside the mobile communication device **500** itself.

At a step **504**, the analysis logic module receives sensor and positioning data of the user from sensor and positioning systems of the mobile communication device **500**. At a step **506**, the analysis logic module stores the sensor and positioning data in a time line format in the annotated sensor data database **502**, i.e. the sensor and positioning data is time stamped to record time and date of each or some of the collected data items. The annotated sensor data database **502** includes sensor and positioning data received from the mobile communication device **500**, annotated with the information from user feedback on corresponding previously identified activities.

At a step **508,** the analysis logic module analyzes the time line and delineates one or more activity periods and recognizes user activities in each activity period using different types of machine learning algorithms, which are trained using the data stored in the annotated sensor data database **502**. For accurately identifying the activities, the analysis logic module may classify the annotated sensor and positioning data based on type of user, type of mobile device, type of user group, type of mobile device group, and demographic factors, etc. to take into account of environments and individual characteristics of the users. For example, acceleration data sensed by an accelerometer of the mobile communication device **500** for an active athletics specialized in long distance running may be different from the acceleration data sensed for a casual runner running very seldom. The active athletics may have longer steps with more predictable frequency between steps with lower variation of the frequency, than the casual runners. Therefore, for similar sensed data, the activity type may differ based on type of user group. Further, some mobile communication devices might have higher accuracy than others, therefore, for similar sensed data, the activity type may differ based on type of mobile communication device. The demographic classification may refer to segmenting users based on their gender, age, place of living etc, as the sensor data for a same activity might vary from person to person. The analysis logic module may further weigh the classified sensor data to take into consideration user specific features i.e. data could have weighted element consisting of average data from all users and user specific element from certain user based on the usage history.

At a step **510**, the analysis logic module may use the classified sensor data to make a time line of the user activities, and communicate the time line to the mobile communication device **500.** The mobile communication device **500** may display the time line on a user interface of the device **500.** At a step **512**, the analysis logic module may receive proposals for correcting one or more user activities in the timeline, from the mobile communication device **500.** The proposals for correcting the user activities may be provided to the annotated sensor data database **502** for training of the machine learning algorithms.

At a step **514,** the mobile communication device **500** may monitor whether or not the proposed time line has been viewed by the user using user interface analytics. If the user has viewed the proposed time line but not provided any correction in any activity of the timeline, then no feedback may be considered as positive feedback and is updated in the annotated sensor data database **502** that the analysis of one or more activities was correct and may be used as a training data for the algorithms. Further, if the user has viewed a storyline/timeline of a certain day and has been active in making corrections, one can conclude that that the recognized activities, which the user has not changed in the storyline, are probably considered to be correct and may be used as a training data for the algorithms. Further, it can be concluded that the recognized activities, which the user has changed in the storyline, are probably not correct. The amount of corrections made by users to certain activity can be used as a measure of recognition accuracy in regard to that activity. In the annotated sensor data database **502**, the sensor data may be labeled, with a certain confidence of the labeling, based on the annotations containing information about user feedback on the corresponding proposed activities.

In an embodiment of the present invention, the analysis logic module may implement a first machine learning algorithm for determining user activities based on the sensor and positioning data, and newly introduce a second machine learning algorithm. However, if the data analyzed based on the second algorithm is more accurate than the data analyzed by the first algorithm, then the second algorithm may be determined to be priority algorithm over the first algorithm. This feature enables to test different setups and get "votes" on the capability of a machine learning algorithm.

It should be noted here that the steps **504** to **514** are only illustrative and other alternatives can also be provided where one or more steps are added, one or more steps are removed, or one or more steps are provided in a different sequence without departing from the scope of the claims herein.

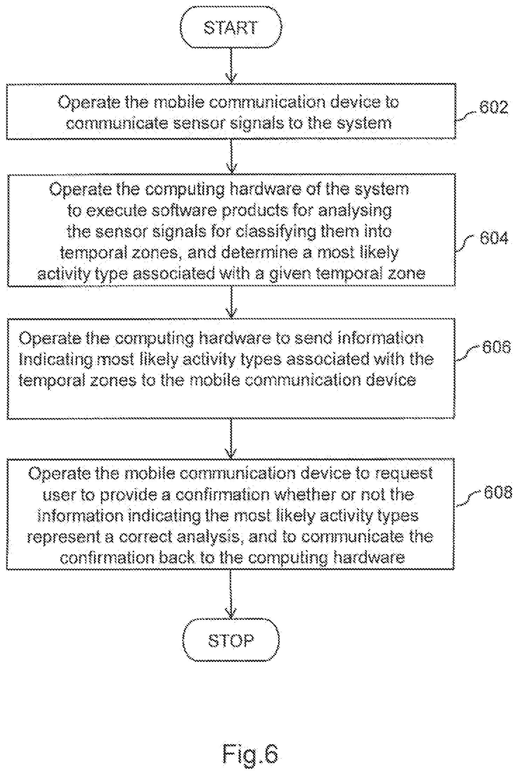

**Fig. 6** is an illustration of steps of using the system **100** for tracking and recording movements of the mobile communication device **102,** in accordance with the present disclosure, and has been explained in conjunction with Figs. 1 and 2. The method is depicted as a collection of steps in a logical flow diagram, which represents a sequence of steps that can be implemented in hardware, software, or a combination thereof.

At a step **602,** the mobile communication device **102** is operable to communicate one or more sensor signals to the server system **104,** wherein the sensor signals are indicative of motion associated with activities to which the mobile communication device **102** is exposed by its user. The sensor signals are output of one or more sensors of the mobile communication device **102.**

At a step **604,** the computing hardware of the server system **104** is operable to execute one or more software products for analyzing the sensor signals to classify them into one or more temporal zones, hereinafter referred to as activity zone, and for analyzing the sensor signals within each activity zone to determine a most likely activity type associated with the activity zone.

At a step **606,** the computing hardware is operated to send information indicating one or more most likely activity types associated with the one or more activity zones to the mobile communication device **102.**

At a step **608,** the mobile communication device **102** is operable to request its user to provide a confirmation whether or not the information indicating the most likely activity types represent a correct analysis, and to communicate the confirmation back to the computing hardware for amending parameters and/or algorithms employed in the software products which execute analysis of the sensor signals to improve their accuracy.

It should be noted here that the steps **602** to **608** are only illustrative and other alternatives can also be provided where one or more steps are added, one or more steps are removed, or one or more steps are provided in a different sequence without departing from the scope of the claims herein.

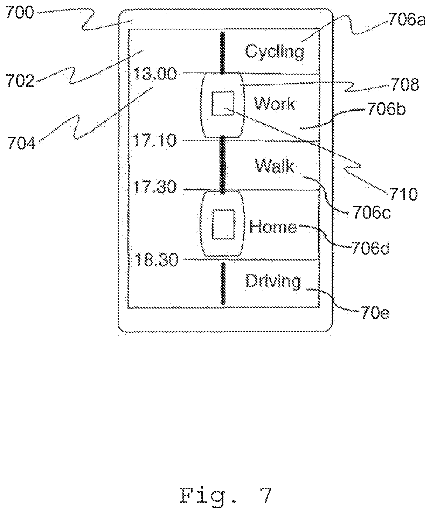

**Fig. 7** is an illustration of a graphical user interface (GUI) **702** of a mobile communication device 700, which is an example of the mobile communication device **102,** and has been explained in conjunction with Fig. 1. The embodiment illustrated in Fig. 7 is similar to the embodiment illustrated in Fig. 4. The GUI **702** displays a time-line **704** that is divided into 'activity' zones/periods **706a, 706b, 706c, 706d** and **706e,** hereinafter collectively referred to as activity zones **706**, based on start and end times of one or more activities. Each activity zone **706** illustrates an activity and corresponding location of the activity. Moreover, each activity zone **706** may be illustrated by a graphical symbol **708** or a text description **710** of the corresponding activity.

In an exemplary embodiment, the timeline **704** indicates that at a time 13:00 pm, a 'cycling' activity of the user ends and he/she is stationed at a 'workplace' until a time 17:10 pm; at the time 17:10 pm, the user starts a 'walking' activity towards home; at a time 17:30 pm, the user reaches home and is at home until a time 18:30 pm; at the time 18:30 pm, the user starts a 'driving' activity.

Fig. 7 is merely an example, which should not unduly limit the scope of the claims herein. One of ordinary skill in the art would recognize many variations, alternatives, and modifications of embodiments herein.

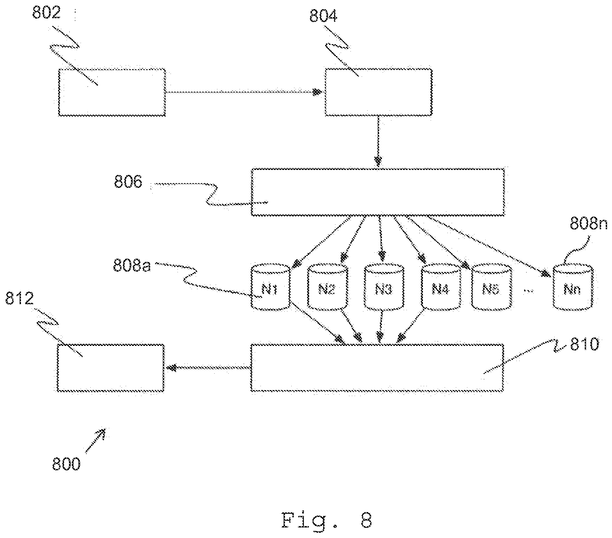

**Fig. 8** is an illustration of an activity analysis system **800**, explained in conjunction with Fig. 1, in accordance with the present disclosure. The activity analysis system **800** identify user activities based on sensor and positioning data of corresponding mobile communication device, and history, profile, demographics, and activity type of user.

In an embodiment of the present invention, the activity analysis system **800** may be present inside a remote server system **104.** In another embodiment of the present invention, the activity analysis system **800** may be present, at least in part, inside the mobile communication device **102** itself.

The activity analysis system **800** includes a receiving module **802**, a pre-processing module **804**, a pre-classification module **806,** a first through nth classifier nodes **808a** till **808n,** hereinafter referred to as classifier nodes **808**, an activity determination module **810**, and an output module **812**. The receiving module **802** collects raw data, i.e. unprocessed data from the sensors **108** and positioning systems **110** of the mobile communication device **102**. The pre-processing module **804** pre-processes the raw data collected by the receiving module **802**. Examples of pre-processing the data include, but are not limited to, filtering the data, performing domain transitions such as time to frequency domain conversion using Fast Fourier Transformation (FFT), classifying the data, averaging the data and combining the data, performing correlations with one or more pre-determined data sets representative of various types of user activities.

The pre-classification module **806** receives the pre-processed data from the pre-processing module **804** and pre-classifies it into one or more broad categories. For example, the sensor data received from a motion sensor of the mobile communication device **102** is compared with a predetermined speed value to differentiate between slow motion, i.e. walking and running stairs, and fast motion i.e. running and cycling, and classify the motion data into broad categories such as 'slow motion' and 'fast motion'. In an embodiment, the pre-classification module **806** includes rule sets and/or predefined deterministic algorithms for pre-classifying the pre-processed data.

Each classifier node **808** includes a processor that is dedicated to identifying characteristics of the sensor data corresponding to a predefined activity. For example, the first classifier node N1 **808a** may be specialized in identifying characteristics of the sensor data pertaining to 'cycling' activity, the second classifier node N2 **808b** may be specialized in identifying 'walking activity', the third classifier node N3 **808c** may be specialized in identifying 'running' activity, and so on.

The classifier nodes **808** are configured to process the pre-classified data substantially in parallel, where each classifier node **808** generates a likelihood of the corresponding predefined activity for the pre-classified data. In an exemplary embodiment, the first classifier node N1 **808a** dedicated to identification of 'cycling' activity may generate a probability value '1', the second classifier node N2 **808b** may generate a probability value '0.3' and the third classifier node N3 **808c** may generate a probability value '0.8', when the user performs a 'cycling' activity.

The activity determination module **810** may aggregate the probability values generated by the classifier nodes **808** to determine an 'activity type' corresponding to the sensor and positioning data collected by the receiving module **802**. In addition to aggregating the probabilities of the classifier nodes **808**, the activity determination module **810** may employ deterministic rules such as transition windows to determine the 'activity type'. The transition window may set inertia to activities in order not to toggle activities too often. For example, it is unlikely that an activity type would change from 'cycling' to 'walking' and back to 'cycling' very fast. The deterministic rules such as transition windows may be implemented using models such as hidden Markov models (HMM) and more complex models based on HMMs.

The output module **812** provides one or more 'activity types' determined by the activity determination module **810** to the mobile communication device **102**. In an embodiment, the output module **812** may display the determined activity types on a graphical user interface (GUI) of the mobile communication device 102 in a timeline format.

Fig. 8 is merely an example, which should not unduly limit the scope of the claims herein. One of ordinary skill in the art would recognize many variations, alternatives, and modifications of embodiments herein.

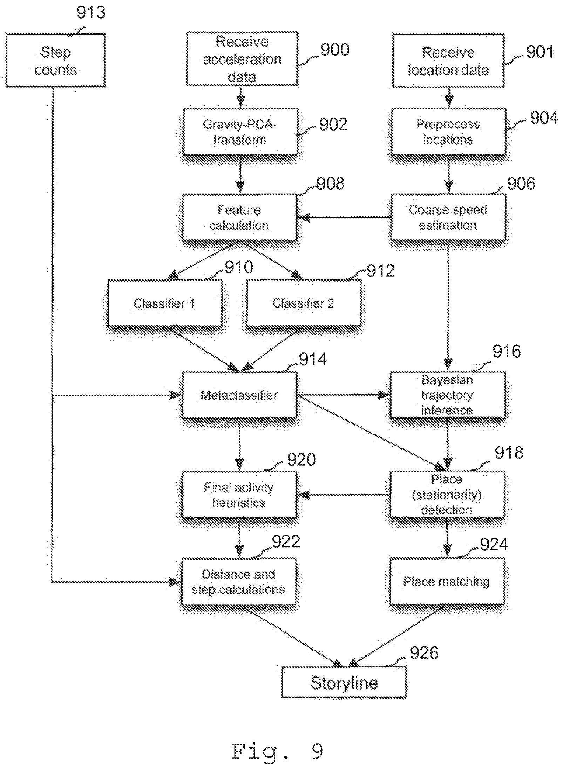

**Fig. 9** is an illustration of steps of a method for identifying a 'cycling' activity of a user based on the sensor and positioning data of the corresponding mobile communication device, in accordance with an embodiment of the present disclosure.

In an embodiment of the present invention, the steps of the method for identifying the 'cycling' activity of a user may be executed by the activity analysis system **800** of Fig. 8. The activity analysis system **800** may be present inside a remote server system 104 or may be present, at least in part, inside the mobile communication device 102 of user itself.

At a step **900**, a set of acceleration data is received from an accelerometer sensor of the mobile communication device **102**. The set of acceleration data is in raw format, i.e. unprocessed, and may be collected every 30 seconds by the accelerometer sensor. In an exemplary embodiment, the set of acceleration data has a duration of 3 seconds and includes acceleration samples collected every 1/20 seconds. Thus, each 3 second data set includes total 60 acceleration samples and each acceleration sample includes acceleration of the mobile communication device **102** in x, y, and z directions, in a coordinate system oriented with the mobile communication device **102.** Thus, each acceleration sample includes three values and the three second data set includes total 180 (3X60) values.

At a step **901,** a set of location data is received from a positioning system of the mobile communication device **102.** The positioning data may include timestamps, location coordinates, and estimated horizontal accuracy from mobile location services. In an exemplary embodiment, the location data is received at intervals ranging from few seconds to few minutes.

At a step **902**, the set of acceleration data undergoes gravity transformation, in which, for each acceleration sample, a new transformed sample is calculated, where corresponding z-component is oriented along a mean value of the acceleration vector. In another embodiment, the set of acceleration data may undergo a principal component analysis (PCA) transformation, in which, for each acceleration sample, a new transformed sample is calculated, where corresponding z-coordinate remains same, but the corresponding x and y components are transformed so that they are oriented along the principal components of the acceleration sample, when only x and y components are included.

At a step **904**, the location data may be pre-processed, where examples of pre-processing the data includes, but are not limited to, filtering the data, performing domain transitions such as time to frequency domain conversion using Fast Fourier Transformation (FFT), classifying the data, averaging the data, performing one or more correlations on the data, and combining the data. At a step **906**, a coarse speed of the mobile communication device 102 may be estimated based on the pre-processed location data. For example, the coarse speed may be estimated based on distance between consecutive location co-ordinates and time difference between the consecutive location co-ordinates.

At a step **908**, one or more features of sensor and location data are estimated based on the transformed acceleration samples and the estimated course speed, where each 'feature' has a numeric value. Examples of features include means, variances, minimums and maximums for each of the (x, y, z) components of the transformed acceleration samples, components of Fourier transformed versions of the x, y, or z components of the acceleration sample, and so forth.

The user activity corresponding to data obtained at the step **908** may be recognized based on a first classification at a step **910** using a first classifier and a second classification at a step **912** using a second classifier. In an embodiment, the first and second classifiers may be similar to classifier nodes **808** of Fig. 8. Although two classifiers are illustrated herein, it would be apparent to a person skilled in the art, that more than two classifiers can be used for recognizing the user activity.

The first and second classifiers at the steps **910** and **912** use standard supervised classification algorithms such as neural network, decision forest or support vector machine for classification. The first classifier is a binary classifier that is trained on a large training data set with training samples classified as 'cycling' or 'non-cycling', and generates an activity label 'cycling' or 'non-cycling' for the data sample obtained at the step **908.** The second classifier is a multiclass classifier that is trained on a smaller set of more accurately labeled data, and generates an activity label from one of 'cycling', 'running', 'car', 'train', 'walking' and 'other' for the data sample obtained at the step **908.**

In an embodiment, the user activity is recognized as 'cycling' if both the first and second classifiers generate activity label as 'cycling' for the data sample obtained at the step **908.** In another embodiment, the user activity is recognized based on the activity label generated by the second classifier when the first classifier generates an activity label as 'not cycling'. In yet another embodiment, the user activity is recognized as 'other', when the first classifier generates an activity label as 'not cycling' and the second classifier generates an activity label as 'cycling'. In yet another embodiment, the first classifier may generate a probability that the user activity is 'not cycling'. When the probability of 'not cycling' is high, then other classifier results indicating 'cycling' as activity might be omitted.

At a step **913,** the step counts of the user are calculated, and at a step **914**, a meta-classifier utilizes the step count data and the data generated by the first and second classifiers to combine activities recognized at the steps **910** and **912** to form one or more activity periods. In an embodiment, when there are N consecutive acceleration samples, such that 1st and last of them are labeled with a given activity, and the majority (or at least x % of them) belong to that activity, the whole period of N consecutive samples is identified as an activity period.

At a step **916,** one or more activity periods may be associated with respective locations based on location data and Bayesian 'interactive multiple models' smoothing algorithm. At a step 918, one or more stationary segments may be recognized based on the location data, when the mobile communication device **102** is stationary and no activity is performed therein.

At a step **920**, final activity heuristics-type analysis takes place based on the processed location and acceleration data. The heuristics are rules, and an example of a rule is: if a stationary segment recognized at the step **918** has a period of no recognized activity shorter than x seconds, and the neighboring activity periods have a duration greater than y seconds, the stationary segment is replaced with an activity period labeling it with the neighboring activity/activities. For example, if in a 10 minutes cycling activity, the user appears to have stopped for 1 minute in between, then the 1 minute stationary segment is ignored, and the whole 10 minutes are associated with cycling activity. Moreover, if in a 10 minutes period, there are consecutive cycling and transport activities, and there is no stopping of at least n minutes in between and no walking activity, then the whole 10 minutes period is labeled with the activity that has happened for majority of the time. However, if there is at least one detected walking sample between transport and cycling activities, then the cycling and transport activities form two different activity periods.

At a step **922**, distance and step calculations are performed, and at a step **924** place matching is performed to optimize the accuracy of user activities recognized at the step 920. Finally, at a step **926**, a storyline is created which includes various user activities in a timeline format.

Fig. 9 is merely an example, which should not unduly limit the scope of the claims herein. One of ordinary skill in the art would recognize many variations, alternatives, and modifications of embodiments herein.

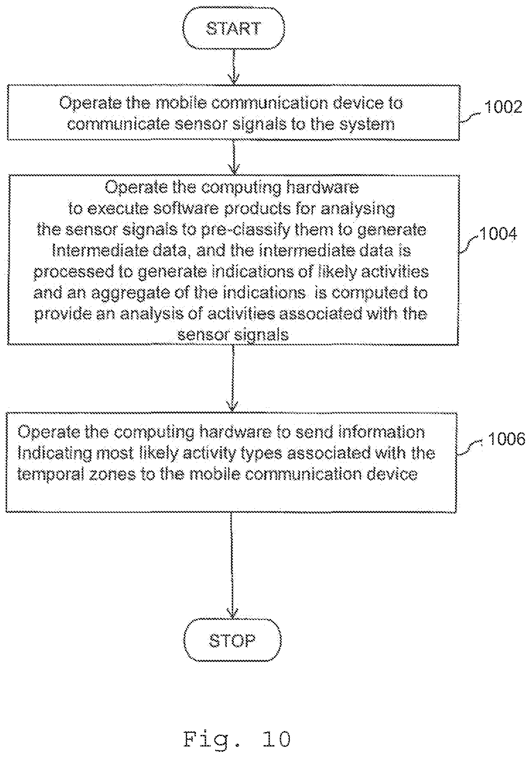

**Fig. 10** is an illustration of steps of using the system **100** for tracking and recording movements of the mobile communication device **102**, in accordance with the present disclosure, and has been explained in conjunction with Figs. 1 and 2. The method is depicted as a collection of steps in a logical flow diagram, which represents a sequence of steps that can be implemented in hardware, software, or a combination thereof.

At a step **1002**, the mobile communication device **102** is operable to communicate one or more sensor signals, or sensor data corresponding thereto, to the server system 104, wherein the sensor signals are indicative of motion associated with activities to which the mobile communication device **102** is exposed by its user. The sensor signals, or the corresponding sensor data, are outputs of one or more sensors of the mobile communication device 102.

At a step **1004**, the computing hardware of the server system **104** is operable to execute one or more software products for analyzing the sensor signals and corresponding sensor data, wherein the computing hardware is operable to pre-classify the sensor signals to generate intermediate data, and the intermediate data is thereafter processed in one or more processors to generate one or more indications of likely activities associated with the sensor signals. The computing hardware is further operable to compute an aggregate of the one or more indications to provide an analysis of activities associated with the sensor signals.

At a step **1006**, the computing hardware is operable to send information indicating most likely activity types associated with the one or more temporal zones to the mobile communication device **102.**

It should be noted here that the steps **1002** to **1006** are only illustrative and other alternatives can also be provided where one or more steps are added, one or more steps are removed, or one or more steps are provided in a different sequence without departing from the scope of the claims herein.

Although embodiments of the present invention have been described comprehensively in the foregoing, in considerable detail to elucidate the possible aspects, those skilled in the art would recognize that other versions of the invention are also possible. Embodiments of the present invention are susceptible to being employed for monitoring prisoners in prisons, for monitoring patients in home for elderly people, in hospitals and such like.

## Claims

1. A method of operating a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, **characterized in that** the method includes:
receiving, by the system, from the at least one mobile communication device one or more sensor signals indicative of motion associated with activities to which the at least one mobile communication device is exposed by its user, wherein a set of location data is received from a positioning system of the mobile communication device (102), wherein the location data includes timestamps and location coordinates from mobile location services,
operating the computing hardware of the system to execute one or more software products for analyzing the one or more sensor signals, wherein the computing hardware is operable to:
pre-classify the one or more sensor signals to generate intermediate data;
process the intermediate data in one or more processors to generate one or more indications of likely activity types associated with the one or more sensor signals;
compute an aggregate of the one or more indications to provide an analysis of one or more activity types associated with the one or more signals;
analyze the one or more sensor signals to classifying them into one or more activity periods, and analyzing the one or more sensor signals within each given activity period to determine a most likely activity type associated with the given activity period;
recognize (918) one or more stationary segments based on the location data, when the mobile communication device (102) is stationary and no activity is performed therein,
determine whether two or more of the activity periods should be replaced with a single activity period based on a heuristics-type analysis based on the determined activity types and a length of time of corresponding activity periods, and, if so, generating at least one activity period replacing the two or more of the activity periods; and
the heuristic type analysis being based on rules, wherein according to a rule, if a recognized stationary segment has a period of no recognized activity shorter than a first predetermined number of seconds, and the neighboring activity periods have a duration greater than a second predetermined number of seconds, the stationary segment is replaced with an activity period labeling it with the neighboring activities;
send information indicating the most likely activity types, and corresponding activity periods and/or replacement activity periods to the at least one mobile communication device;
at the at least one mobile communication device, a graphical user interface, GUI (302), displaying a summary of activities of the user received from the system (204) in a time line format;
the system comprising classifier nodes, each classifier node (808) including a processor that is dedicated to identifying characteristics of the sensor data corresponding to a predefined activity.

2. A method as claimed in Claim 1, **characterized in that** the method further includes
operating the at least one mobile communication device to request its user to provide a confirmation whether or not the information indicating the one or more most likely activity types associated with the one or more activity periods represent a correct analysis, and to communicate the confirmation back to the computing hardware for amending parameters and/or algorithms employed in the one or more software products which execute analysis of the one or more sensor signals to improve their accuracy.

3. A method as claimed in Claims 1 or 2, **characterized in that** the method includes configuring the one or more processors to process the one or more sensor signals substantially in parallel, wherein the one or more processors are mutually specialized in identifying characteristics of the one or more signals corresponding to one or more activity types to which the one or more processors are dedicated.

4. A method as claimed in any of Claims 1 to 3, **characterized in that** the method includes operating the system to generate a temporal log of activities experienced by the at least one mobile communication device.

5. A method as claimed in any of Claims 1 to 4, **characterized in that** the method includes implementing at least one mobile communication device by way of at least one of: a personal computer, a portable media device, a smart phone, a wrist-worn phone, a phablet, a mobile telephone, a tablet computer.

6. A method as claimed in any of Claims 1 to 5, **characterized in that** the method includes implementing the one or more sensors of the at least mobile communication device using at least one of: a gyroscopic angular sensor, an accelerometer, a GPS position sensor, a cellular position sensor, a magnetometer, a microphone, a camera, a temperature sensor.

7. A method as claimed in any of Claims 1 to 6, **characterized in that** the one or more software products are operable to implement when executed on the computing hardware at least one of:
a supervised or semisupervised classification analysis of information included in the one or sensor signals; and
a heuristics analysis of information included in the one or more sensor signals.

8. A method as claimed in Claim 7, **characterized in that** the supervised or semisupervised classification algorithms can use as input the amplitudes of the frequency components of the information included in the one or more sensor signals, and the output of the classification algorithms are estimated probabilities of different activity types, conditional on the sensor signals.

9. A method as claimed in any of Claims 1 to 8, wherein sending information indicating the most likely activity types, and corresponding activity periods and/or replacement activity periods comprises sending analyzed activity results to the at least one mobile device for display on a graphical user interface of a software application of the at least one mobile communication device to present the sent analyzed activity results in a form of at least one timeline, wherein different analyzed activity types are represented by a plurality of mutually different symbols in respect of the at least one time line.

10. A system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, comprising: one or more processors; and at least one memory coupled to the processors and comprising instructions executable by the processors, the processors operable when executing the instructions to perform a method according to any of Claims 1 to 9.

11. One or more computer-readable non-transitory storage media embodying software that is operable when executed to perform a method according to any of Claims 1 to 9.

12. A storage media as claimed in Claim 11, **characterized in that** the software is downloadable from a software application store to a mobile communication device.

13. A mobile communication device configured to be implemented in the system according to Claim 10 and in use for executing a method according to any of Claims 1 to 9.

14. A computer-implemented method comprising:
transmitting, by at least one mobile communication device via a communication network, one or more sensor signals indicative of motion associated with activities to which the at least one mobile communication device is exposed by its user to a system for tracking and recording movements of at least one mobile communication device, the system being operated according to a method of one of claims 1 to 9;
receiving at the at least one mobile communication device information indicating for the transmitted one or more sensor signals one or more most likely activity types;
displaying a summary of the one or more most likely activity types on a touch screen display of the one or more mobile communication devices in a time-line format comprising at least one graphical activity indication symbol, wherein the touch screen display is configured to:
present a pop-up menu in case of detecting a user touching or pointing the graphical activity indication symbol;
the pop-up menu including a text field and symbol field, configured for enabling the user to modify the activity type and graphical activity indication symbol; and
communicating a corresponding modification to the system.

15. The method of Claim 14, comprising at least one of the following features:
- the timeline format further comprises a graphical location symbol, and wherein the touch screen display is further configured to present a pop-up menu in case of detecting a user touching or pointing the graphical location symbol, the pop-up menu configured for enabling the user to modify the location;
- the received information indicating the one or more most likely activity types generated according to a method according to at least one of claims 1 to 9, or generated by a system according to claim 10;
- the sensor signals generated by one or more sensors selected from the group comprising: a gyroscopic angular sensor, an accelerometer, a GPS position sensor, a cellular position sensor, a magnetometer, a microphone, a camera, a temperature sensor;
- transmitting the one or more sensor signals comprises transmitting the sensor signals continuously or in a buffered format;
- transmitting the one or more sensor signals comprises transmitting the sensor signals in raw format or in a processed format generated by the mobile communication device before transmitting the sensor data to the system.

## Patentansprüche

1. Verfahren zum Betreiben eines Systems zum Verfolgen und Aufzeichnen von Bewegungen von mindestens einer mobilen Kommunikationsvorrichtung, die einen oder mehrere Bewegungssensoren und eine drahtlose Schnittstelle umfasst, wobei das System ein Kommunikationsnetzwerk zum Kommunizieren mit der mindestens einen mobilen Kommunikationsvorrichtung und eine Rechner-Hardware zum Verarbeiten von Daten, die im Betrieb von der mindestens einen mobilen Kommunikationsvorrichtung geliefert werden, umfasst, **dadurch gekennzeichnet, dass** das Verfahren umfasst:
Empfangen, durch das System, eines oder mehrerer Sensorsignale, die eine Bewegung anzeigen, die Aktivitäten zugeordnet ist, denen die mindestens eine mobile Kommunikationsvorrichtung durch ihren Benutzer ausgesetzt wird, von der mindestens einen mobilen Kommunikationsvorrichtung, wobei ein Satz von Standortdaten von einem Positionierungssystem der mobilen Kommunikationsvorrichtung (102) empfangen wird, wobei die Standortdaten Zeitstempel und Ortskoordinaten von mobilen Ortungsdiensten umfassen,
Betreiben der Rechner-Hardware des Systems, um ein oder mehrerer Softwareprodukte zum Analysieren der einen oder mehreren Sensorsignale auszuführen, wobei die Rechenhardware betreibbar ist, um:
das eine oder die mehreren Sensorsignale vorab zu klassifizieren, um Zwischendaten zu erzeugen;
die Zwischendaten in einem oder mehreren Prozessoren zu verarbeiten, um ein oder mehrere Hinweise auf wahrscheinliche Aktivitätstypen zu erzeugen, die dem einem oder den mehreren Sensorsignalen zugeordnet sind;
eine Gesamtsumme der einen oder mehreren Hinweise zu berechnen, um eine Analyse von einer oder mehreren Aktivitätstypen bereitzustellen, die dem einen oder den mehreren Signalen zugeordnet sind;
das eine oder die mehreren Sensorsignale zu analysieren, um sie in eine oder mehrere Aktivitätsperioden zu klassifizieren und das eine oder die mehreren Sensorsignale innerhalb jeder gegebenen Aktivitätsperiode zu analysieren, um einen wahrscheinlichsten Aktivitätstyp zu bestimmen, der der gegebenen Aktivitätsperiode zugeordnet ist;
ein oder mehrere stationäre Segmente basierend auf den Standortdaten zu erkennen (918), wenn die mobile Kommunikationsvorrichtung (102) stationär ist und keine Aktivität darin ausgeführt wird,
zu bestimmen, basierend auf einer heuristischen Analyse, die auf den bestimmten Aktivitätstypen und einer Zeitdauer von entsprechenden Aktivitätsperioden basiert, ob zwei oder mehr der Aktivitätsperioden durch eine einzige Aktivitätsperiode ersetzt werden sollen, und, wenn ja, mindestens eine Aktivitätsperiode zu erzeugen, die die zwei oder mehr Aktivitätsperioden ersetzt; und
wobei die heuristische Typenanalyse auf Regeln basiert, wobei gemäß einer Regel, wenn ein erkanntes stationäres Segment eine Periode ohne erkannte Aktivität, die kürzer als eine erste vorbestimmte Anzahl von Sekunden ist, aufweist und die benachbarten Aktivitätsperioden eine Dauer aufweisen, die größer als eine zweite vorbestimmte Anzahl von Sekunden ist, das stationäre Segment durch eine Aktivitätsperiode ersetzt wird, die mit den benachbarten Aktivitäten markiert wird;
Information, die die wahrscheinlichsten Aktivitätstypen und entsprechenden Aktivitätsperioden und/oder Ersatzaktivitätsperioden anzeigen, an die mindestens eine mobile Kommunikationsvorrichtung zu senden;
an der mindestens einen mobilen Kommunikationsvorrichtung, einer grafischen Benutzeroberfläche, GUI, (302), eine von dem System (204) empfangene Zusammenfassung der Aktivitäten des Benutzers in einem Zeitlinienformat anzuzeigen;
wobei das System Klassifizierungsknoten umfasst, wobei jeder Klassifizierungsknoten (808) einen Prozessor umfasst, der dazu bestimmt ist, Merkmale der Sensordaten zu identifizieren, die einer vordefinierten Aktivität entsprechen.

2. Verfahren nach Anspruch 1, **dadurch gekennzeichnet, dass** das Verfahren ferner das Betreiben der mindestens einen mobilen Kommunikationsvorrichtung umfasst, um ihren Benutzer aufzufordern, eine Bestätigung darüber abzugeben, ob die Information, die die eine oder die mehreren am wahrscheinlichsten Aktivitätstypen anzeigen, die der einen oder den mehreren Aktivitätsperioden zugeordnet sind, eine korrekte Analyse darstellen, und die Bestätigung an die Rechner-Hardware zurückzumelden, um Parameter und/oder Algorithmen zu ändern, die in dem einen oder den mehreren Softwareprodukten verwendet werden, die eine Analyse des einen oder der mehreren Sensorsignale durchführen, um ihre Genauigkeit zu verbessern.

3. Verfahren nach Anspruch 1 oder 2, **dadurch gekennzeichnet, dass** das Verfahren das Konfigurieren des einen oder der mehreren Prozessoren umfasst, um das eine oder die mehreren Sensorsignale im Wesentlichen parallel zu verarbeiten, wobei der eine oder die mehreren Prozessoren gemeinsam darauf spezialisiert sind, Merkmale des einen oder der mehreren Signale zu identifizieren, die einem oder mehreren Aktivitätstypen entsprechen, denen die einen oder mehreren Prozessoren zugeordnet sind.

4. Verfahren nach einem der Ansprüche 1 bis 3, **dadurch gekennzeichnet, dass** das Verfahren das Betreiben des Systems umfasst, um ein zeitliches Protokoll der Aktivitäten zu erzeugen, die durch die mindestens eine mobile Kommunikationsvorrichtung wahrgenommen werden.

5. Verfahren nach einem der Ansprüche 1 bis 4, **dadurch gekennzeichnet, dass** das Verfahren das Implementieren umfasst von mindestens einer mobilen Kommunikationsvorrichtung durch mindestens eines von: einem Personalcomputer, einer tragbaren Medienvorrichtung, einem Smartphone, einem am Handgelenk getragenen Telefon, einem Phablet, einem Mobiltelefon, einem Tablet-Computer.

6. Verfahren nach einem der Ansprüche 1 bis 5, **dadurch gekennzeichnet, dass** das Verfahren das Implementieren des einen oder der mehreren Sensoren der mindestens einen mobilen Kommunikationsvorrichtung umfasst, wobei mindestens eines verwendet wird von: einem gyroskopischen Winkelsensor, einem Beschleunigungssensor, einem GPS-Positionssensor, einem zellularen Positionssensor, einem Magnetometer, einem Mikrofon, einer Kamera, einem Temperatursensor.

7. Verfahren nach einem der Ansprüche 1 bis 6, **dadurch gekennzeichnet, dass** das eine oder die mehreren Softwareprodukte betriebsfähig sind, um bei Ausführung auf der Rechner-Hardware mindestens eines zu implementieren von:
einer überwachten oder teilüberwachten Klassifizierungsanalyse der in dem einen oder den mehreren Sensorsignalen umfassten Information; und
eine heuristische Analyse von Information, die in dem einem oder den mehreren Sensorsignalen umfasst ist.

8. Verfahren nach Anspruch 7, **dadurch gekennzeichnet, dass** die überwachten oder teilüberwachten Klassifizierungsalgorithmen die Amplituden der Frequenzkomponenten der in dem einem oder den mehreren Sensorsignalen umfassten Information als Eingabe verwenden können und die Ausgabe der Klassifizierungsalgorithmen geschätzte Wahrscheinlichkeiten verschiedener Aktivitätstypen sind, abhängig von den Sensorsignalen.

9. Verfahren nach einem der Ansprüche 1 bis 8, wobei das Senden von Information, die die am wahrscheinlichsten Aktivitätstypen und entsprechende Aktivitätsperioden und/oder Ersatz-Aktivitätsperioden anzeigt, das Senden von analysierten Aktivitätsergebnissen an die mindestens eine mobile Vorrichtung zur Anzeige auf einer grafischen Benutzeroberfläche einer Softwareanwendung der mindestens einen mobilen Kommunikationsvorrichtung umfasst, um die gesendeten analysierten Aktivitätsergebnisse in Form von mindestens einer Zeitachse darzustellen, wobei verschiedene analysierte Aktivitätsarten durch eine Vielzahl von voneinander unterschiedlichen Symbolen in Bezug auf die mindestens eine Zeitleiste dargestellt werden.

10. System zum Verfolgen und Aufzeichnen von Bewegungen von mindestens einer mobilen Kommunikationsvorrichtung, die einen oder mehrere Bewegungssensoren und eine drahtlose Schnittstelle umfasst, das System umfassend ein Kommunikationsnetzwerk zum Kommunizieren mit der mindestens einen mobilen Kommunikationsvorrichtung und eine Rechner-Hardware zum Verarbeiten von Daten, die im Betrieb von der mindestens einen mobilen Kommunikationsvorrichtung geliefert werden, umfassend: einen oder mehrere Prozessoren; und mindestens einen Speicher, der mit den Prozessoren gekoppelt ist und Anweisungen umfasst, die von den Prozessoren ausgeführt werden können, wobei die Prozessoren beim Ausführen der Anweisungen betreibbar sind, um ein Verfahren nach einem der Ansprüche 1 bis 9 auszuführen.

11. Ein oder mehrere computerlesbare, nicht-flüchtige Speichermedien, die Software enthalten, die, wenn sie ausgeführt wird, funktionsfähig ist, um ein Verfahren nach einem der Ansprüche 1 bis 9 durchzuführen.

12. Speichermedium nach Anspruch 11, **dadurch gekennzeichnet, dass** die Software von einem Softwareanwendungsspeicher auf eine mobile Kommunikationsvorrichtung heruntergeladen werden kann.

13. Mobile Kommunikationsvorrichtung, die dazu konfiguriert ist, in dem System nach Anspruch 10 implementiert zu werden und zur Ausführung eines Verfahrens nach einem der Ansprüche 1 bis 9 verwendet zu werden.

14. Computerimplementiertes Verfahren, umfassend:
Senden, durch mindestens eine mobile Kommunikationsvorrichtung über ein Kommunikationsnetz, eines oder mehrerer Sensorsignale, die eine Bewegung anzeigen, die Aktivitäten zugeordnet ist, denen die mindestens eine mobile Kommunikationsvorrichtung durch ihren Benutzer ausgesetzt wird, an ein System zum Verfolgen und Aufzeichnen von Bewegungen von mindestens einer mobilen Kommunikationsvorrichtung, wobei das System gemäß einem Verfahren nach einem der Ansprüche 1 bis 9 betrieben wird;
Empfangen, an der mindestens einen mobilen Kommunikationsvorrichtung, von Information, die für das eine oder die mehreren gesendeten Sensorsignale eine oder mehrere am wahrscheinlichsten Aktivitätstypen anzeigen;
Anzeigen einer Zusammenfassung der einen oder mehreren am wahrscheinlichsten Aktivitätstypen auf einer Touchscreen-Anzeige der einen oder der mehreren mobilen Kommunikationsvorrichtungen in einem Zeitachsenformat, das mindestens ein grafisches Aktivitätsanzeigesymbol umfasst, wobei die Touchscreen-Anzeige dazu konfiguriert ist, um:
ein Popup-Menü darzustellen, wenn ein Benutzer erkannt wird, der das grafischen Aktivitätsanzeigesymbol berührt oder darauf zeigt;
wobei das Popup-Menü ein Textfeld und ein Symbolfeld umfasst, die so konfiguriert sind, dass der Benutzer den Aktivitätstyp und das grafische Aktivitätsanzeigesymbol ändern kann; und
Kommunizieren einer entsprechenden Änderung an das System.

15. Verfahren nach Anspruch 14, umfassend mindestens eines der folgenden Merkmale:
das Zeitachsenformat umfasst ferner ein grafisches Standortsymbol, und wobei die Touchscreen-Anzeige ferner dazu konfiguriert ist, ein Popup-Menü anzuzeigen, falls ein Benutzer erkannt wird, der das grafische Standortsymbol berührt oder darauf zeigt, wobei das Popup-Menü dazu konfiguriert ist, dem Benutzer zu ermöglichen, den Standort zu ändern;
die empfangene Information zeigt die eine oder die mehreren am wahrscheinlichsten Aktivitätstypen an, die gemäß einem Verfahren nach mindestens einem der Ansprüche 1 bis 9 oder gemäß einem System nach Anspruch 10 erzeugt werden;
die Sensorsignale werden von einem oder mehreren Sensoren erzeugt, die aus der Gruppe ausgewählt sind, die umfasst: einen gyroskopischen Winkelsensor, einen Beschleunigungssensor, einen GPS-Positionssensor, einen zellularen Positionssensor, ein Magnetometer, ein Mikrofon, eine Kamera, einen Temperatursensor;
das Senden des einen oder der mehreren Sensorsignale umfasst, die Sensorsignale kontinuierlich oder in einem gepufferten Format zu senden;
das Senden des einen oder der mehreren Sensorsignale umfasst, die Sensorsignale in einem rohen Format oder in einem verarbeiteten Format, das von der mobilen Kommunikationsvorrichtung vor dem Senden der Sensordaten an das System erzeugt wird, zu senden.

## Revendications

1. Procédé de fonctionnement d'un système pour suivre et enregistrer des mouvements d'au moins un dispositif de communication mobile comprenant un ou plusieurs capteurs de mouvement et une interface sans fil, le système comprenant un réseau de communication pour communiquer avec l'au moins un dispositif de communication mobile et un matériel informatique pour traiter des données fournies en fonctionnement à partir de l'au moins un dispositif de communication mobile, **caractérisé en ce que** le procédé comprend:
recevoir, par le système, à partir de l'au moins un dispositif de communication mobile un ou plusieurs signaux de capteur indicatifs d'un mouvement associé à des activités auxquelles l'au moins un dispositif de communication mobile est exposé par son utilisateur, un ensemble de données d'emplacement étant reçu à partir d'un système de localisation du dispositif de communication mobile (102), les données d'emplacement comprenant des estampilles temporelles et des coordonnées d'emplacement à partir de services de localisation mobiles,
amener le matériel informatique du système à exécuter un ou plusieurs produits logiciels pour analyser le ou les signaux de capteur, le matériel informatique étant fonctionnel pour:
classifier préalablement le ou les signaux de capteur pour générer des données intermédiaires;
traiter les données intermédiaires dans un ou plusieurs processeurs pour générer une ou plusieurs indications de types d'activités probables associés au ou aux signaux de capteur;
calculer un agrégat de la ou des indications pour fournir une analyse d'un ou plusieurs types d'activités associés au ou aux signaux;
analyser le ou les signaux de capteur pour les classifier en une ou plusieurs périodes d'activité, et analyser le ou les signaux de capteur à l'intérieur de chaque période d'activité donnée pour déterminer un type d'activités le plus probable associé à la période d'activité donnée;
reconnaître (918) un ou plusieurs segments stationnaires sur la base des données d'emplacement, lorsque le dispositif de communication mobile (102) est stationnaire et qu'aucune activité n'est effectuée dans celui-ci,
déterminer si au moins deux des périodes d'activité doivent être remplacées par une période d'activité unique sur la base d'une analyse de type heuristique basée sur les types d'activités déterminés et une longueur de temps des périodes d'activité correspondantes, et, si tel est le cas, générer au moins une période d'activité remplaçant les au moins deux périodes d'activité; et
l'analyse de type heuristique étant basée sur des règles, dans laquelle, selon une règle, si un segment stationnaire reconnu a une période d'activité non reconnue plus courte qu'un premier nombre prédéterminé de secondes, et que les périodes d'activité voisines ont une durée plus grande qu'un second nombre prédéterminé de secondes, le segment stationnaire est remplacé par une période d'activité en l'étiquetant aux activités voisines;
envoyer des informations indiquant les types d'activités les plus probables, et les périodes d'activité correspondantes et/ou les périodes d'activité de remplacement à l'au moins un dispositif de communication mobile;
afficher, au niveau de l'au moins un dispositif de communication mobile, par une interface utilisateur graphique, GUI, (302), un résumé d'activités de l'utilisateur reçues à partir du système (204) dans un format de ligne de temporelle;
le système comprenant des noeuds de classification, chaque noeud de classification (808) comprenant un processeur qui est dédié à identifier des caractéristiques des données de capteur correspondant à une activité prédéfinie.

2. Procédé tel que revendiqué à la revendication 1, **caractérisé en ce que** le procédé comprend en outre
amener l'au moins un dispositif de communication mobile à demander à son utilisateur de fournir une confirmation si oui ou non les informations indiquant le ou les types d'activités les plus probables associés à la ou aux périodes d'activité représentent une analyse correcte, et à communiquer en retour la confirmation au matériel informatique pour modifier des paramètres et/ou des algorithmes utilisés dans le ou les produits logiciels qui exécutent une analyse du ou des signaux de capteur pour améliorer leur précision.

3. Procédé tel que revendiqué à la revendication 1 ou 2, **caractérisé en ce que** le procédé comprend
configurer le ou les processeurs pour traiter le ou les signaux de capteur sensiblement en parallèle, le ou les processeurs étant mutuellement spécialisés dans l'identification de caractéristiques du ou des signaux correspondant à un ou plusieurs types d'activités auxquels le ou les processeurs sont dédiés.

4. Procédé tel que revendiqué à l'une quelconque des revendications 1 à 3, **caractérisé en ce que** le procédé comprend amener le système à générer un journal temporel d'activités subies par l'au moins un dispositif de communication mobile.

5. Procédé tel que revendiqué à l'une quelconque des revendications 1 à 4, **caractérisé en ce que** le procédé comprend mettre en oeuvre au moins un dispositif de communication mobile au moyen d'au moins l'un parmi: un ordinateur personnel, un dispositif multimédia portable, un téléphone intelligent, un téléphone porté au poignet, une phablette, un téléphone mobile, une tablette électronique.

6. Procédé tel que revendiqué à l'une quelconque des revendications 1 à 5, **caractérisé en ce que** le procédé comprend mettre en oeuvre le ou les capteurs de l'au moins un dispositif de communication mobile en utilisant au moins l'un parmi: un capteur angulaire gyroscopique, un accéléromètre, un capteur de position GPS, un capteur de position cellulaire, un magnétomètre, un microphone, une caméra, un capteur de température.

7. Procédé tel que revendiqué à l'une quelconque des revendications 1 à 6, **caractérisé en ce que** le ou les produits logiciels sont aptes à mettre en oeuvre, lorsqu'ils sont exécutés sur le matériel informatique, au moins l'un parmi:
une analyse de classification supervisée ou semi-supervisée d'informations incluses dans le ou les signaux de capteur; et
une analyse heuristique d'informations incluses dans le ou les signaux de capteur.

8. Procédé tel que revendiqué à la revendication 7, **caractérisé en ce que** les algorithmes de classification supervisés ou semi-supervisés peuvent utiliser en tant qu'entrée les amplitudes des composants de fréquence des informations incluses dans le ou les signaux de capteur, et les sorties des algorithmes de classification sont des probabilités estimées de différents types d'activités, conditionnées aux signaux de capteur.

9. Procédé tel que revendiqué à l'une quelconque des revendications 1 à 8, dans lequel l'envoi d'informations indiquant les types d'activités les plus probables, et les périodes d'activité correspondantes et/ou les périodes d'activité de remplacement comprend l'envoi de résultats d'activités analysées à l'au moins un dispositif mobile pour un affichage sur une interface utilisateur graphique d'une application logicielle de l'au moins un dispositif de communication mobile pour présenter les résultats d'activités analysées envoyés sous la forme d'au moins une ligne temporelle, différents types d'activités analysées étant représentés par une pluralité de symboles mutuellement différents par rapport à l'au moins une ligne temporelle.

10. Système pour suivre et enregistrer des mouvements d'au moins un dispositif de communication mobile comprenant un ou plusieurs capteurs de mouvement et une interface sans fil, le système comprenant un réseau de communication pour communiquer avec au moins un dispositif de communication mobile et un matériel informatique pour traiter des données fournies en fonctionnement à partir de l'au moins un dispositif de communication mobile, comprenant: un ou plusieurs processeurs; et au moins une mémoire couplée aux processeurs et comprenant des instructions exécutables par les processeurs, les processeurs étant fonctionnels, lorsqu'ils exécutent les instructions, pour réaliser un procédé selon l'une quelconque des revendications 1 à 9.

11. Support(s) de stockage non transitoire(s) lisible(s) par ordinateur incorporant un logiciel qui est apte, lorsqu'il est exécuté, à réaliser un procédé selon l'une quelconque des revendications 1 à 9.

12. Supports de stockage selon la revendication 11, **caractérisé en ce que** le logiciel est téléchargeable à partir d'un magasin d'applications logicielles sur un dispositif de communication mobile.

13. Dispositif de communication mobile configuré pour être mis en oeuvre dans le système selon la revendication 10 et en utilisation pour exécuter un procédé selon l'une quelconque des revendications 1 à 9.

14. Procédé mis en oeuvre par ordinateur comprenant:
émettre, par au moins un dispositif de communication mobile par l'intermédiaire d'un réseau de communication, un ou plusieurs signaux de capteur indicatifs d'un mouvement associé à des activités auxquelles l'au moins un dispositif de communication mobile est exposé par son utilisateur à un système pour suivre et enregistrer des mouvements d'au moins un dispositif de communication mobile, le système étant commandé conformément à un procédé selon l'une des revendications 1 à 9;
recevoir, au niveau de l'au moins un dispositif de communication mobile, des informations indiquant, pour le ou les signaux de capteur émis, un ou plusieurs types d'activités les plus probables;
afficher un résumé du ou des types d'activités les plus probables sur un dispositif d'affichage à écran tactile du ou des dispositifs de communication mobile dans un format de ligne temporelle comprenant au moins un symbole d'indication d'activité graphique, le dispositif d'affichage à écran tactile étant configuré pour:
présenter un menu en incrustation dans le cas d'une détection du fait qu'un utilisateur touche ou pointe le symbole d'indication d'activité graphique;
le menu en incrustation comprenant un champ de texte et un champ de symbole, configurés pour permettre à l'utilisateur de modifier le type d'activités et le symbole d'indication d'activité graphique; et
communiquer une modification correspondante au système.

15. Procédé selon la revendication 14, comprenant au moins l'une des caractéristiques suivantes:
le format de ligne temporelle comprend en outre un symbole d'emplacement graphique, et le dispositif d'affichage à écran tactile étant en outre configuré pour présenter un menu en incrustation dans le cas d'une détection du fait qu'un utilisateur touche ou pointe le symbole d'emplacement graphique, le menu en incrustation étant configuré pour permettre à l'utilisateur de modifier l'emplacement;
les informations reçues indiquant le ou les types d'activités les plus probables sont générées selon un procédé selon au moins l'une des revendications 1 à 9, ou générées par un système selon la revendication 10;
les signaux de capteur sont générés par un ou plusieurs capteurs choisis parmi le groupe comprenant: un capteur angulaire gyroscopique, un accéléromètre, un capteur de position GPS, un capteur de position cellulaire, un magnétomètre, un microphone, une caméra, un capteur de température;
l'émission du ou des signaux de capteur comprend l'émission des signaux de capteur de manière continue ou dans un format mis en mémoire tampon;
l'émission du ou des signaux de capteur comprend l'émission des signaux de capteur dans un format brut ou dans un format traité généré par le dispositif de communication mobile avant l'émission des données de capteur au système.
